# EUROPEAN PATENT APPLICATION

(11) **EP 2 335 674 A1**
(43) Date of publication of application: **22.06.2011**
(21) Application number: 09015679.5
(22) Date of filing: 18.12.2009
(51) Int. Cl.: A61K 8/04, A61K 8/31, A61K 8/34, A61Q 5/06

(54) **Hair styling emulsion foam**

(71) Applicant: KPSS-Kao Professional Salon Services GmbH, 64297 Darmstadt (DE)
(72) Inventor: Schmidt, Sabine, 65462 Ginsheim-Gustavsburg (DE); Weichaus, Dirk, 64404 Bickenbach (DE)
(74) Representative: Grit, Mustafa

(57) **Abstract**

Present invention relates to a hair styling emulsion foam which has excellent setting, shine enhancing and volumizing and bodyfiying effects even it is applied in comparatively small quantities. Accordingly, the object of the present invention is an aqueous emulsion foam composition comprising at least one oil and/or a wax or their mixture, at least one emulsifier, at least one adipic acid ester and at least propellant.

## Description

Present invention relates to a hair styling emulsion foam which has excellent setting, shine enhancing and volumizing and bodyfiying effects even it is applied in comparatively small quantities.

Hair styling compositions have been known used commonly with variable purposes. There is a wide range of products available on the market and their number is increasing daily. Basic benefits expected by the users are setting, making hair manageable, diminish flyaways, allow restyling and styled hair must feel as natural as possible, and very importantly, not reducing shine and these should be achieved with relatively small amount of product applied onto hair which must therefore be excellently spreadable onto hair. Although, some of the aforementioned properties have already been addressed, there is still highly need of improvements.

The inventors have surprisingly found out that an aqueous emulsion foam composition comprising at least one oil and/or a wax or their mixture, at least one emulsifier, at least one adipic acid ester and at least propellant has excellent foam properties from foam appearance point of view, effective in small quantities, makes hair manageable, and has excellent volumizing and bodifying effects. The hair feels excellently natural both visually and upon touching and looks excellently shiny. It has furthermore observed that foam composition is excellently spreadable onto hair.

Thus, the object of the present invention is an aqueous emulsion foam composition comprising at least one oil and/or a wax or their mixture, at least one emulsifier, at least one adipic acid ester and at least propellant.

Another object of the present invention is the use of an aqueous emulsion foam composition comprising at least one oil and/or a wax or their mixture, at least one emulsifier, at least one adipic acid ester and at least propellant for styling hair.

Still another subject of the present invention is a process for styling hair wherein an aqueous emulsion foam composition comprising at least one oil and/or a wax or their mixture, at least one emulsifier, at least one adipic acid ester and at least propellant is applied onto wet or dry hair and hair is optionally dried without rinsing off the composition.

Emulsion foam composition of the present invention comprises at least one oil and/or wax or mixtures thereof. In principal any oily and or way compound suitable for use in a cosmetic composition is suitable for the purposes of the present invention. Preferably oil is selected from either natural or synthetic ones. Suitable non-limiting ones are avocado oil, coconut oil, palm oil, sesame oil, peanut oil, whale oil, sunflower oil, almond oil, peach kernel oil, wheat germ oil, macadamia nut oil, night primrose oil, jojoba oil, castor oil, or also olive oil, and soya oil.

Synthetic ones may be mineral oils such as paraffin oil and petrolatum as well as fatty alcohol fatty acid esters such as isopropyl myristate, palmitate, stearate and isostearate, oleyl oleate, isocetyl stearate, hexyl laurate, myristyl myristate, oleyl erucate, polyethylene glycol and polyglyceryl fatty acid esters, cetyl palmitate, etc, and also silicone oils, both volatile and non-volatile ones, such as dimethciones as known from Dow Corning with DC 200 series as well as arylated silicones such as phenyl methicone, phenyl trimethicone, diphenyl dimethicone, diphenylsiloxy phenyl trimethicone, tetramethyl tetraphenyl trisiloxane, triphenyl trimethicone, tetramethyl tetraphenyl trisiloxane and trimethyl pentaphenyl trisiloxane, and also aminated silicones such as amodimethcone and its derivatives and also cyclic silicones such as cyclomethicone, cylopentasiloxane and cyclohexasiloxane.

With the term wax it is meant, any hydrophobic compound which is solid at room temperature. Suitable examples are fatty alcohols according to the general structure

R₂₀-OH

where R₂₀ is a saturated, branched or non-branched fatty alkyl chain with 12 - 24 C atoms such as lauryl alcohol, myristyl alcohol, palmityl alcohol, cetyl alcohol, stearyl alcohol, behenyl alcohol and their mixtures, vaseline, petrolatum, beeswax, candelila wax, Carnauba wax and microcrystalline wax.

Concentration of at least one oil and/or wax or their mixture is in the range of 1 to 25%, preferably 1 to 20%, more preferably 2 to 15% and most preferably 2.5 to 15% by weight calcualted to total composition excluding propellant. The concentration ranges mentioned here are the total concentration of the compounds in case more than one compound is used.

Emulsion foam composition of the present invention comprises at least one emulsifier.

As a rule any emulsifier is suitable for the purposes of the present invention, however, non-ionic emulsifiers especially ethoxylated fatty alcohols and monoglycerides especially in mixture are the most preferred ones.

Preferred and most suitable nonionic surfactants useful in the compositions according to invention are C₁₀-C₂₂-fatty alcohol ethoxylates. Suitable non-limiting examples are oleth-10, oleth-11, oleth-12, oleth-15, oleth-16, oleth-20, oleth-25, oleth-30, oleth-35, oleth-40, laureth-10, laureth-11, laureth-12, laureth-13, laureth-15, laureth-16, laureth-20, laureth-25, laureth-30, laureth-35, laureth-40, laureth-50, ceteth-10, ceteth-12, ceteth-14, ceteth-15, ceteth-16, ceteth-17, ceteth-20, ceteth-25, ceteth-30, ceteth-40, ceteth-45, cetoleth-10, cetoleth-12, cetoleth-14, cetoleth-15, cetoleth-16, cetoleth-17, cetoleth-20, cetoleth-25, cetoleth-30, cetoleth-40, cetoleth-45, ceteareth-10, ceteareth-12, ceteareth-14, ceteareth-15, ceteareth-16, ceteareth-18, ceteareth-20, ceteareth-22, ceteareth-25, ceteareth-30, ceteareth-40, ceteareth-45, ceteareth-50, isosteareth-10, isosteareth-12, isosteareth-15, isosteareth-20, isosteareth-22, isosteareth-25, isosteareth-50, steareth-10, steareth-11, steareth-14, steareth-15, steareth-16, steareth-20, steareth-25, steareth-30, steareth-40, steareth-50, steareth-80 and steareth-100. Additional examples of similar compounds can be found in the cosmetic ingredient dictionaries and cosmetic textbooks.

Again most preferred non-ionic surfactants are monogylcerides and their ethoxylated and or propoxylated derivatives and their mixtures. Suitable non-limiting examples are glyceryl monostearate, glyceryl monolaurate, glyceryl monomyristate glyceryl monooleate, glyceryl monopalmitate, glyceryl monobehenate, and polyalkyleneglycol ether of fatty acid glyceride or partial glyceride with at least 30 polyalkylene units are with 30 to 1000, preferably 30 to 500, more preferably 30 to 200 and most preferably 40 to 100 polyethyleneglycol units. Examples to latter are PEG-30 hydrogenated castor oil, PEG-35 hydrogenated castor oil, PEG-40 hydrogenated castor oil, PEG-45 hydrogenated castor oil, PEG-50 hydrogenated castor oil, PEG-55 hydrogenated castor oil, PEG-60 hydrogenated castor oil, PEG-65 hydrogenated castor oil, PEG-80 hydrogenated castor oil, PEG-100 hydrogenated castor oil, PEG-200 hydrogenated castor oil, PEG-35 castor oil, PEG-50 castor oil, PEG-55 castor oil, PEG-60 castor oil, PEG-80 castor oil, PEG-100 castor oil, PEG-200 castor oil. Additional examples of similar compounds can be found in the cosmetic ingredient dictionaries and cosmetic textbooks.

In a preferred embodiment of the present invention, emulsion foam compositions of the present invention comprises at least one ethoxylated fatty alcohol and at least one monoglyceride as emulsifiers and preferably at a weight ratio of 10:1 1 to 1:10, preferably 5:1 to 1:5, more preferably 2:1 to 1:2 and most preferably 1:1.

Further suitable non-ionic surfactants, especially useful in mixture with the above mentioned preferred non-ionic surfactants, are alkyl polyglucosides of the general formula

R₇ - O - (R₈O)ₙ O - Zₓ

wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5. Examples are decyl polyglucoside, cocoyl polyglucoside both are commercially available.

Further, nonionic surfactants may be useful, again especially in mixture with above mentioned most preferred non-ionic surfactants are long-chain fatty acid mono- and dialkanolamides, such as coco fatty acid mono and di ethanolamide and myristic fatty acid mono and di ethanolamide.

In the same way, additionally useful nonionic surfactants are, preferably in mixture with the above mentioned most preferred non-ionic surfactants, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Cationic surfactants are also suitable as emulsifiers especially the ones according to the general formula 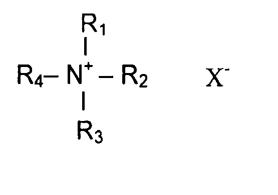 where R₁S a saturated or unsaturated, branched or non-branched alkyl chain with 8-22 C atoms or

R₅CO NH (CH₂)n

where R₅ is saturated or unsaturated, branched or non-branched alkyl chain with 7-21 C atoms and n has typical value of 0 - 4 or

R₆CO O (CH₂)n

where R₆ is saturated or unsaturated, branched or non-branched alkyl chain with 7-21 C atoms and n has typical value of 0 - 4, and
and R₂, R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate, especially in mixture with the above mentioned most preferred non-ionic surfactants.

Non-limiting examples to suitable cationic surfactants are cetyl trimethyl ammonium chloride, myristoyl trimethyl ammonium chloride, behentrimonium chloride, trimethyl cetyl ammonium bromide, stearyl trimethyl ammonium chloride, dimethyl stearyl ammonium chloride, stearamidopropyldimethyl ammonium chloride.

As further surfactant component, the compositions according to the invention may also contain amphoteric or zwitterionic surfactants as emulsifiers in mixture with the above mentioned mist preferred surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and ―acetate.

Further, suitable within the meaning of the present invention are, especially in mixture with the above mentioned most preferred non-ionic surfactants, anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono-, di- or tri alkyl phosphates.

Additional anionic surfactants useful are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₉- (C₂H₄O)ₙ- O - CH₂COOX,

wherein R₉ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Concentration of one or more emulsifier in the compositions of the present invention is in the range of 0.1 to 25%, preferably 0.5 to 20%, more preferably 1 to 17.5% and most preferably 1 to 15% by weight calculated to total composition, excluding propellant. The concentration ranges mentioned here are the total surfactant concentrations in case the composition comprises more than one surfactant.

Emulsion foam compositions of the present invention comprise at least one adipic acid ester. Suitable ones are according to the general structure 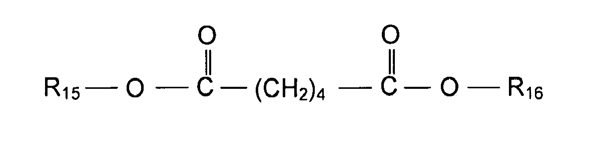 wherein R15 and R16 are same or different an H or an alkyl chain which may be saturated or unsaturated, branched or straight with 8 to 24C atoms, preferably 12 to 22C atoms which may as well be substituted, and Bis-diglyceryl polyacyl adipate-1 and Bis-diglyceryl polyacyl adipate-2.

Suitable non-limiting examples are dicetyl adipate, diisodecyl adipate, ethylhexyl adipate, distearyl adipate, dihexyldecyl adipate, dioctyl adipate, Bis-diglyceryl polyacyl adipate-1 and Bis-diglyceryl polyacyl adipate-2. Preferred are Bis-diglyceryl polyacyl adipate-1 and Bis-diglyceryl polyacyl adipate-2 and the most preferred is Bis-diglyceryl polyacyl adipate-2.

Concentration of at least one adipic acid ester in the compositions of the present invention is in the range of 0.1 to 25%, preferably 0.5 to 20%, more preferably 1 to 17.5% and most preferably 1 to 15% by weight calculated to total composition, excluding propellant. The concentration ranges mentioned here are the total surfactant concentrations in case the composition comprises more than one surfactant.

Emulsion foam composition comprises at least one propellant at a concentration of 1 to 20%, preferably 2 to 15%, more preferably 2 to 12.5% and most preferably 2 to 10% by weight clauclated to total composition. Suitable propellants are alkanes including haloalkanes such as ethane, propane, n-butane, isobutene, pentane, isopentane, chlorodifluoromethane, 1-chloro-1,1-diflorocarbon, 1,1,1,2-tetrafluoroethane and1,1-difluroethane and dimethyl ether. Preferred are alkynes and most preferred are propane, n-butane and their mixtures.

It has been observed that the bulk density of the foam is somehow unique to the emulsion compositions of the present invention and varies between 0.1 and 0.2 g/mL, preferably between 0.11 and 0.17g/mL and more preferably between 0.12 and 0.15 g/mL measured at ambient temperature immediately after release from the aerosol container.

Compositions of the present invention comprise preferably polyethyleneglycol and/or polypropyleneglycol. It has been observed that presence of these compounds improve product stability. The concentration can vary between 1 and 30%, preferably 1 and 25% more preferably 2 and 20% and most preferably 3 and 15% by weight calculated to total composition excluding propellant.

Suitable non-limiting examples to polyethyleneglycols and polypropyleneglycols are PEG-4, PEG-6, PEG-7, PEG-8, PEG-9, PEG-10, PEG-12, PEG-14, PEG-16, PEG-18, PEG-20, PEG-32, PEG-33, PEG-40, PEG-45, PEG-55, PEG-60, PEG-75, PEG-80, PEG-90, PEG-100, PEG-135, PEG-150, PEG-180, PEG-180, PEG-200, PEG-220, PEG-240, PEG-350, PEG-400, PEG-450, PEG-500, PEG-800, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-180M, PPG-3, PPG-7, PPG-9, PPG-12, PPG-13, PPG-15, PPG-16, PPG-17, PPG-20, PPG-26, PPG-30, PPG-33, PPG-34, PPG-51, PPG-52, and PPG-69. Preferred are PEG-10, PEG-12, PEG-14, PEG-16, PEG-18, PEG-20, PEG-32, PEG-33, PEG-40, PEG-45, PEG-55, PEG-60, PEG-75, PEG-80, PEG-90, PEG-100, PEG-135, PEG-150, PPG-12, PPG-13, PPG-15, PPG-16, PPG-17, PPG-20, PPG-26, PPG-30, PPG-33, PPG-34, PPG-51, PPG-52, and PPG-69. More preferred are PEG-12, PEG-14, PEG-16, PEG-18, PEG-20, PEG-32, PEG-33, PEG-40, PEG-45, PEG-55, PEG-60, PEG-75, PEG-80, PEG-90, PEG-100, PPG-15, PPG-16, PPG-17, PPG-20, PPG-26, PPG-30, PPG-33, and PPG-34. Most preferred are PEG-14, PEG-16, PEG-18, PEG-20, PEG-32, PEG-33, PEG-40, PEG-45, PEG-55, PEG-60, PEG-75, PEG-80, PPG-20, PPG-26, PPG-30, PPG-33, and PPG-34.

Compositions of the present invention can comprise at least one styling polymer at a concentration of 0.1 to 25%, preferably 0.2 to 20%, more preferably 0.5 to 15 and most preferably 1 to 15% by weight calculated to total composition excluding propellant.

Within the meaning of the present invention at least one styling polymer is selected from the anionic, non-ionic, cationic and/or amphoteric or zwitterionic ones. It should be understood that cationic polymers function at the same time as conditioners.

Suitable non-ionic polymer is first of all vinylpyrrolidon polymers either homopolymers or copolymers with, especially, vinylacetate. Those are known with the trade name "Luviskol" as homopolymers Luviskol K 30, K 60 or K 90 as well copolymers Luviskol VA 55, VA 64, Plus from BASF AG and advantage LS-E from ISP.

Natural non-ionic polymers are as well suitable for the composition of the present invention. Those are such as cellulose, chitosan, guar gum, neutralised shellac and their derivatives.

As amphoteric polymers which can be used alone or in mixture with at least one additional nonionic polymer, reference is here made in particular to copolymers of N-octyl acrylamide, (meth)acrylic acid and tert.-butyl aminoethyl methacrylate of the type "Amphomer®"; copolymers from methacryloyl ethyl betaine and alkyl methacrylates of the type "Yukaformer®", e.g.. the butyl methacrylate copolymer "Yukaformer® Am75"; copolymers from monomers containing carboxyl groups and sulfonic groups, e.g.. (meth)acrylic acid and itaconic acid, with monomers such as mono- or dialkyl aminoalkyl (meth)acrylates or mono- or dialkyl aminoalkyl (meth)-acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199.

Suitable anionic polymers alone or in combination with non-ionic polymers are vinyl - alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g.. "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof of the type "Reten®"; etc.

Further suitable anionic polymers are Acrylate copolymers available under trade name Salcare SC 81, PEG/PPG 25/25 dimethicone / acrylate copolymer available under trade name Luviglex Silk from BASF, Acrylates/t-butylacrylamide copolymer available under trade name Ultrahold Strong, Advantage LC-E which is vinylcaprolactam/PVP/dimethylaminoethylmethacrylate copolymer and VA/crotonates copolymer available under trade name Luviset CA 66.

Cationic polymers as styling agents and/or conditioners are comprised advantageously in the emulsion foam compositions of the present invention. Suitable examples are the ones known with the INCI name Polyquaternium such as Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46, Polyquaternium 24, Polyquaternium 67, Polyquaternium 68, Polyquaternium 72 and Polyquaternium 87.

Emulsion foam compositions of the present invention may comprise at least one thickening polymer. Any polymer known for thickening aqueous and/or aqueous-alcoholic medium is suitable within the meaning of the present invention. Preferred are anionic polymers such as acrylates and or alkyl acrylated, non-ionic polymers such as cellulose and its derivatives ethyl cellulose, hydroxyetyhlcellulose, guar gum such as hydroxyl propyl guar gum, and xanthan gum. Preferred are cellulose and guar gum and their derivatives and most preferred is hydroxyethylcellulose.

Concentration of thickening polymer is in the range of 0.05 to 2%, preferably 0.05 to 1.5 and more preferably 0.1 to 1% by weight calculated to total composition excluding propellant.

Foam composition of the present invention comprises at least one alcohol such as ethanol, isopropanol, propoanol etc. Most preferred is ethanol. Concentration of ethanol is in the range of 1 to 25%, preferably 2.5 to 20%, more preferably 5 to 20% and most preferably 5 to 15% by weight calculated to total composition excluding propellants.

The compositions according to the invention may also comprise further agents, such as proteins, for example bamboo protein, and protein hydrolyzates and polypeptides, e.g. keratin hydrolyzates, collagen hydrolyzates of the type "Nutrilan^{®}" or elastin hydrolyzates, as well as, in particular vegetable, optionally cationized protein hydrolyzates, for example "Gluadin^{®}".

Additional natural plant extracts can as well form part of the compositions of the present invention. Those are incorporated usually in an amount of about 0.01 % to about 5 %, preferably 0.05 % to 3.5 %, in particular 0.1 % to 2 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, coconut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon^{®}" products, "Herbasol^{R}", "Sedaplant^{R}" and "Hexaplant^{R}". Extracts and the preparation thereof are also described in "Hagers Handbuch der pharmazeutischen Praxis". 4^{th} Ed..

Foam compositions of the present invention may contain UV filters either for stabilization of the product colour and/or for protection of hair from environmental influences such as loss of elasticity, loss of hair colour (bleaching effect of sun light). Suitable UV-absorbing substance are Polysilicone-15, 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxy- benzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzophenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzylidenebornane-2-one and the salts thereof and/or 3-(4'-methyl benzylidene)-DL-campher, and/or polysiliocne-15.

The suitable amount of the UV-absorber ranges from about 0.01 % to 1% by weight, calculated to the total composition. Attention should be paid to the stability and solubility especially when using UV filter as salts, e.g. anionic UV filter salts.

In a further embodiment of the present invention, the compositions comprise at least one direct dye for colouring hair. Suitable direct dyes are cationic, anionic, neutral dyes and their mixtures as available commercially from various suppliers and used mainly in semipermanent hair coloration.

Non-limiting examples to cationic dyes are Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Further suitable direct dyes are anionic dye. Suitable non-limiting examples are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Further suitable dyes for colouring hair within the meaning of the present invention are those of neutral nitro dyes. Suitable non-limiting examples are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used as hair colorant within the meaning of the present invention for example henna (red or black), alkanna root, laccaic acid, indigo, logwood powder, madder root and rhubarb powder, etc.

It should be noted that the above dyestuffs are also suitable for use in mixture. When using direct dyes of various categories, their compatibility must be checked.

The above mentioned dyestuffs are also used especially the anionic ones for product colouring at reduced concentrations.

Concentration of direct dyes in the compositions of the present invention is within the range of 0.001 to 5%, preferably 0.01 to 3% and more preferably 0.05 to 2%, and most preferably 0.1 to 1% by weight calculated to total composition, calculated to total composition.

Composition of the present invention may comprise at least one dipeptide. The dipeptide compounds according to the present invention comprise two amino acid moieties. In principal, any dipeptide available either natural or synthetic is suitable for the purposes of the present invention. The synthetic ones are preferred. In one of the preferred embodiment of the present invention the amino acid moeities of dipeptide are selected from arginine, tyrosine, valine, tryptophan, alanine, cysteine, glycine, lysine, proline, hydroxyproline and histidine. The dipetides according to the present invention may certainly be of two different amino acids but at the same time two of the same amino acids.

Non-limiting examples to the suitable dipeptides are the ones commercially available and known with their INCI name as Dipeptide-1, Dipeptide-2, Dipeptide-3, Dipeptide-4, Dipeptide-5, Dipeptide-6, Dipeptide-7, Dipeptide-8, and carnosine. The most preferred is carnosine and is of ß-alanin and L-histidine.

Concentration of at least one dipeptide is in the range of 0.01 to 5%, preferably 0.05 to 3% and more preferably 0.1 to 2.5% and most preferably 0.2 to 1.5% by weight calculated to the total composition, excluding propellant.

Furthermore compositions of the present invention can comprise all substances customarily found in such preparations. Examples of such substances are chelating agents, preservatives, fragrances, etc.

The following examples are to illustrate the invention but not to limit.

### Example 1

| | % by weight |
|---|---|
| Stearyl alcohol | 10.0 |
| Glyceryl stearate | 7.5 |
| Bis diglyceryl polyacryl adipate | 10.0 |
| Water | q.s to 100 % |

The above composition was prepared by emulsifying and homogenizing the ingredient at around 70°C and cooling down to ambient temperature.

The above composition was filled into an aerosol can with 94% by weight, bulk, 6% by weight propane/butane, all values are calculated to total composition. The foam had a density of approximately 0.142 g/mL at ambient temperature.

The composition was applied onto freshly washed hair. It was observed that hair body and shine was improved.

### Example 2

| | % by weight |
|---|---|
| Petrolatum | 10.0 |
| Glyceryl stearate | 7.5 |
| Bis diglyceryl polyacryl adipate | 10.0 |
| Water | q.s to 100% |

The above composition was prepared by emulsifying and homogenizing the ingredient at around 70°C and cooling down to ambient temperature.

The above composition was filled into an aerosol can with 94% by weight, bulk, 6% by weight propane/butane, all values are calculated to total composition.

The composition was applied onto freshly washed hair. It was observed that hair body and shine was improved.

### Example 3

| | % by weight |
|---|---|
| VP/VA copolymer | 5.0 |
| Stearyl alcohol | 10.0 |
| Glyceryl stearate | 7.5 |
| Bis diglyceryl polyacryl adipate | 10.0 |
| PEG-32 | 10.0 |
| Water | q.s to 100 % |

The above composition was filled into an aerosol can with 94% by weight, bulk, 6% by weight propane/butane, all values are calculated to total composition.

The above composition had excellent setting and manageability effect when applied onto freshly washed and dried hair. It was observed that spreadability was judged to be excellent and small quantities of the composition was enough to observe the above effects.

### Example 4

| | % by weight |
|---|---|
| VP/VA copolymer | 5.0 |
| Stearyl alcohol | 10.0 |
| Glyceryl stearate | 4.0 |
| Ceteareth-20 | 4.0 |
| Bis diglyceryl polyacryl adipate | 10.0 |
| PEG-32 | 10.0 |
| Benzophenone-3 | 0.5 |
| Water | q.s to 100 % |

The above composition was filled into an aerosol can with 94% by weight, bulk, 6% by weight propane/butane, all values are calculated to total composition.

The above composition had excellent setting and manageability effect when applied onto freshly washed and dried hair. It was observed that spreadability was judged to be excellent and small quantities of the composition was enough to observe the above effects.

### Example 5

| | % by weight |
|---|---|
| VPNA copolymer | 5.0 |
| Stearyl alcohol | 10.0 |
| Glyceryl stearate | 4.0 |
| Ceteareth-20 | 4.0 |
| Bis diglyceryl polyacryl adipate | 10.0 |
| PEG-32 | 10.0 |
| Benzophenone-3 | 0.5 |
| Ethanol | 10.0 |
| Water | q.s to 100% |

The above composition was filled into an aerosol can with 94% by weight, bulk, 6% by weight propane/butane, all values are calculated to total composition.

The above composition had excellent setting, manageability and bodifying effects when applied onto freshly washed and dried hair. It was observed that spreadability was judged to be excellent and small quantities of the composition was enough to observe the above effects. It was furthermore observed that hair shine was not diminished.

### Example 6

| | % by weight |
|---|---|
| VP/VA copolymer | 5.0 |
| Dimethicone | 8.0 |
| Glyceryl stearate | 4.0 |
| Ceteareth-20 | 4.0 |
| Bis diglyceryl polyacryl adipate | 10.0 |
| PEG-32 | 10.0 |
| Benzophenone-3 | 0.5 |
| Ethanol | 10.0 |
| Water | q.s to 100% |

The above composition was filled into an aerosol can with 94% by weight, bulk, 6% by weight propane/butane, all values are calculated to total composition.

The above composition had excellent setting, manageability and bodifying effects when applied onto freshly washed and dried hair. It was observed that spreadability was judged to be excellent and small quantities of the composition was enough to observe the above effects. It was furthermore observed that hair shine was improved.

### Example 7

| | % by weight |
|---|---|
| VP/VA copolymer | 5.0 |
| Stearyl alcohol | 10.0 |
| Glyceryl stearate | 4.0 |
| Ceteareth-20 | 4.0 |
| Bis diglyceryl polyacryl adipate | 10.0 |
| PEG-32 | 10.0 |
| Benzophenone-3 | 0.5 |
| Ethanol | 10.0 |
| Water | q.s to 100 % |

The above composition was filled into an aerosol can with 94% by weight, bulk, 3% by weight propane/butane and 3% by weight dimethylether, all values are calculated to total composition.

The above composition had excellent setting, manageability and bodifying effects when applied onto freshly washed and dried hair. It was observed that spreadability was judged to be excellent and small quantities of the composition was enough to observe the above effects. It was furthermore observed that hair shine was not diminished.

### Example 7

| | % by weight |
|---|---|
| VP/VA copolymer | 5.0 |
| Stearyl alcohol | 10.0 |
| Glyceryl stearate | 4.0 |
| Ceteareth-20 | 4.0 |
| Bis diglyceryl polyacryl adipate | 10.0 |
| PEG-32 | 10.0 |
| Benzophenone-3 | 0.5 |
| Ethanol | 10.0 |
| Basic red 51 | 0.25 |
| Basic red 76 | 0.05 |
| Basic yellow 87 | 0.07 |
| Water | q.s to 100% |

The above composition was filled into an aerosol can with 94% by weight, bulk, 6% by weight propane/butane, all values are calculated to total composition.

The above composition had excellent setting, manageability and bodifying effects when applied onto freshly washed and dried hair. It was observed that spreadability was judged to be excellent and small quantities of the composition was enough to observe the above effects. It was furthermore observed that hair shine was not diminished.

## Claims

1. An aqueous emulsion foam composition for hair **characterized in that** it comprises at least one oil and/or a wax or their mixture, at least one emulsifier, at least one adipic acid ester and at least propellant.

2. Composition according to claim 1 **characterized in that** it comprises at least one oil and/or wax at a concentration of 1 to 25% by weight calculated to total composition excluding propellant.

3. Composition according to claims 1 and 2 **characterised in that** it comprises at least one non-ionic emulsifier, preferably selected from fatty alcohol ethoxylates and monoglycerides and their mixtures.

4. Composition according to any of the preceding claims **characterised in that** adipic acid ester is selected from compound according to general structure 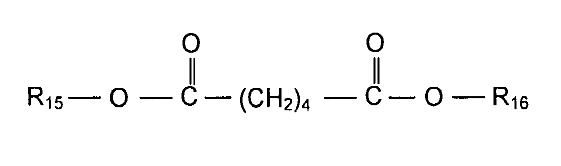 wherein R15 and R16 are same or different an H or an alkyl chain which may be saturated or unsaturated, branched or straight with 8 to 24C atoms, preferably 12 to 22C atoms which may as well be substituted, and Bis-diglyceryl polyacyl adipate-1 and Bis-diglyceryl polyacyl adipate-2.

5. Composition according to claim 5 **characterised in that** adipic acid ester is Bis-diglyceryl polyacyl adipate-1 and Bis-diglyceryl polyacyl adipate-2.

6. Composition according to any of the preceding claims **characterised in that** it comprises at least one adipic acid ester at a concentration of 0.1 to 25% by weight calculated to total composition excluding propellant.

7. Composition according to any of the preceding claims **characterised in that** at least one propellant is selected from alkanes, haloalkanes and dimethyl ether.

8. Composition according to any of the preceding claims **characterised in that** at least one propellant is present at a concentration of 1 to 20% by weight calculated to total composition,

9. Composition according to any of the preceding claims **characterised in that** the foam has a bulk density between 0.1 and 0.2 g/ml measured immediately after releasing from aerosol container at ambient temperature.

10. Composition according to any of the preceding claims **characterised in that** at least one UV filter.

11. Composition according to any of the preceding claims **characterised in that** it comprises polyethyleneglycol and/or polypropyleneglycol, preferably at a concentration of 1 to 30% by weight calculated to total composition excluding propellant.

12. Composition according to any of the preceding claims **characterized in that** it comprises at least one film forming polymer.

13. Composition according to any of the preceding claims **characterized in that** it comprises one or more compounds selected from organic solvent, natural plant extract, direct dye and dipeptide.

14. Use of a composition according to claims 1 to 13 for styling hair.

15. Process for styling hair wherein a composition according to claims 1 to 13 is applied onto wet or dry hair andf hair is optionally dried without rinsing off the composition.
